(19) Europäisches Patentamt
European Patent Office
Office européen des brevets

(11) Veröffentlichungsnummer: **0 027 952**
**B1**

(12) # EUROPÄISCHE PATENTSCHRIFT

(45) Veröffentlichungstag der Patentschrift: **13.04.83**

(51) Int. Cl.³: **C 07 C 125/073,** C 07 C 125/065, C 07 D 307/82

(21) Anmeldenummer: **80106247.2**

(22) Anmeldetag: **15.10.80**

(54) **Verfahren zur Herstellung von N,O-disubstituierten Urethanen.**

(30) Priorität: **27.10.79 DE 2943480**

(43) Veröffentlichungstag der Anmeldung: **06.05.81 Patentblatt 81/18**

(45) Bekanntmachung des Hinweises auf die Patenterteilung: **13.04.83 Patentblatt 83/15**

(84) Benannte Vertragsstaaten: **AT BE CH DE FR GB IT LI NL SE**

(56) Entgegenhaltungen:
**EP-A-0 018 581**
**EP-A-0 018 583**
**EP-A-0 018 588**
**US-A-2 806 051**

(73) Patentinhaber: **BAYER AG, Zentralbereich Patente, Marken und Lizenzen, D-5090 Leverkusen 1, Bayerwerk (DE)**

(72) Erfinder: **Heitkämper, Peter, Dr., Fliederweg 14, D-4047 Dormagen 11 (DE)**
Erfinder: **König, Klaus, Dr., Heymannstrasse 10, D-5090 Leverkusen 1 (DE)**
Erfinder: **Fauss, Rudolf, Dr., Gerstenkamp 10, D-5000 Köln 80 (DE)**
Erfinder: **Findeisen, Kurt, Dr., In der Follmuehle 10, D-5068 Odenthal 2 (DE)**

**EP 0 027 952 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von N,O-disubstituierten Urethanen

Die vorliegende Erfindung betrifft ein verbessertes Verfahren zur Herstellung von Urethanen durch Umsetzung von primären Aminen und Alkoholen mit Carbonylgruppen aufweisenden organischen Verbindungen, wobei als Carbonylgruppen aufweisende organische Verbindungen bestimmte, nachstehend näher beschriebene N-unsubstituierte Urethane und gegebenenfalls N-mono- oder N,N'-disubstituierte Harnstoffe bzw. Polyharnstoffe verwendet werden.

N,O-disubstituierte Urethane entstehen bekanntlich u. a. durch Umsetzung von organischen Isocyanaten mit Alkoholen. Diese Umsetzung ist reversibel, d. h. die Urethane können thermisch in das ihnen zugrundeliegende Isocyanat und dem ihnen zugrundeliegenden Alkohol gespalten werden (vgl. z. B. US-PS 2 409 712). Thermisch in Isocyanate spaltbare Urethane sind daher interessante Ausgangsmaterialien zur Herstellung dieser Isocyanate, die bislang weltweit in größtem Umfang durch Umsetzung von primären Aminen mit Phosgen hergestellt werden. Die phosgenfreie Herstellung von Urethanen und deren anschließende thermische Spaltung stellt eine interessante Alternative zu der genannten großtechnischen Isocyanatherstellung dar. Eine Methode zur phosgenfreien Herstellung von Urethanen besteht in der Umsetzung von Harnstoff mit Aminen und Alkohol (vgl. z. B. US-PS 2 409 712 oder US-PS 2 806 051). Diese Methoden des Standes der Technik sind jedoch mit dem Nachteil behaftet, daß die Urethane in nur unzureichender Ausbeute und in stark mit Nebenprodukten verunreinigter Form erhalten werden.

Es war daher die Aufgabe der vorliegenden Erfindung, ein neues Verfahren zur Herstellung von Urethanen durch Umsetzung von primären Aminen und Alkoholen mit organischen Carbonylverbindungen zur Verfügung zu stellen, welches die Herstellung von N,O-disubstituierten Urethanen in guter Ausbeute und in von unerwünschten Nebenprodukten freier Form gestattet.

Überraschenderweise wurde nunmehr gefunden, daß diese Aufgabe durch die Verwendung von nachstehend näher beschriebenen N-unsubstituierten Urethanen, sowie gegebenenfalls unter zusätzlicher Verwendung von nachstehend näher beschriebenen N-mono- oder N,N'-disubstituierten Harnstoffen bzw. Polyharnstoffen als organische Carbonylverbindungen gelöst werden kann.

Gegenstand der vorliegenden Erfindung ist somit ein Verfahren zur Herstellung von Urethanen der allgemeinen

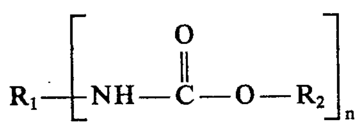

in welcher

$R_1$ für einen gegebenenfalls substituierten aliphatischen Kohlenwasserstoffrest mit 1–18 Kohlenstoffatomen, einen gegebenenfalls substituierten cycloaliphatischen Kohlenwasserstoffrest mit 3 bis 18 Kohlenstoffatomen, einen gegebenenfalls substituierten aromatischen Kohlenwasserstoffrest mit 6–15 Kohlenstoffatomen, einen gegebenenfalls substituierten araliphatischen Kohlenwasserstoffrest mit 7 bis 14 Kohlenstoffatomen, oder für einen gegebenenfalls substituierten 5- oder 6gliedrigen heterocyclischen Rest steht, der zusätzlich noch mit einem Benzolring anelliert sein kann,

$R_2$ für einen gegebenenfalls substituierten Alkylrest mit 1 bis 20 Kohlenstoffatomen, einen gegebenenfalls substituierten Cycloalkylrest mit 3 bis 16 Kohlenstoffatomen und einen gegebenenfalls substituierten Aralkylrest mit 7 bis 14 Kohlenstoffatomen und

n eine ganze Zahl von 1 bis 3 bedeuten,

wobei im Falle von $n = 2$ oder 3 zwischen 2 mit dem Rest $R_1$ verknüpften Urethangruppen mindestens 2 Kohlenstoffatome angeordnet sind,

durch Umsetzung von

a) primären Aminen der Formel

$R_1(NH_2)_n$ und

b) Alkoholen der Formel

$R_2-OH$

mit Carbonylgruppen aufweisenden organischen Verbindungen im Temperaturbereich von 120 bis 350°C, dadurch gekennzeichnet, daß man als Carbonylgruppen aufweisende organische Verbindungen

c) Urethane der Formel

$R_3-O-CO-NH_2$

in welcher

$R_3$ entweder der Definition von $R_2$ entspricht, wobei $R_2$ und $R_3$ gleiche oder verschiedene Reste bedeuten, oder für einen gegebenenfalls Chlor- oder $C_1-C_4$-Alkyl-substituierten aromatischen Kohlenwasserstoffrest mit insgesamt 6 bis 15 Kohlenstoffatomen steht, und gegebenenfalls

d) gegebenenfalls Urethan- oder primäre Aminoendgruppen aufweisende N-mono- oder N,N'-disubstituierte Harnstoffe oder lineare Harnstoffe eines maximalen Molekulargewichts von 2000, deren Harnstoff-, Urethan- bzw. Aminogruppen über Kohlenwasserstoffreste miteinander verknüpft sind oder deren Harnstoffgruppen mit Kohlenwasserstoffresten substituiert sind, die dem Rest $R_1$ entsprechen, und deren gegebenenfalls vorliegende endständigen Urethangruppen mit dem dem als Ausgangsmaterial eingesetzten Alkohol entsprechenden Rest $R_2$ substituiert sind, und deren Gesamtgehalt an Harnstoffgruppen $-NH-CO-NH-$ und Urethangruppen $-NH-CO-O-$ einem Gehalt an für beide Gruppen charakteristischen Struktureinheiten $-NH-CO-$ zwischen 5 und 58 Gew.-% entspricht,

verwendet, wobei sowohl (i) die Herstellung von Aryl-mono- und/oder -polyurethanen durch Umsetzung von gegebenenfalls substituierten O-Alkyl-, O-Cycloalkyl- oder O-Aralkylcarbamidsäureestern mit primären aromatischen mono- und/oder Polyaminen in Gegenwart von Alkoholen ohne gleichzeitige Mitverwendung der unter d) genannten Ausgangsverbindungen als auch (ii) die Herstellung von aliphatischen und/oder cycloaliphatischen Di- und/oder Polyurethanen durch Umsetzung von Carbamidsäureestern mit primären aliphatischen und/oder cycloaliphatischen Di- und/oder Polyaminen und Alkoholen in Abwesenheit der unter d) genannten Ausgangsverbindungen ausgenommen sind.

Die veröffentlichte europäische Patentanmeldung 80 102 185.8 (0 018 588) beschreibt die Herstellung von aliphatischen und/oder cycloaliphatischen Di- und/oder Polyurethanen durch Umsetzung von Carbamidsäureestern mit primären aliphatischen und/oder cycloaliphatischen Di- und/oder Polyaminen in Gegenwart von Alkoholen und gegebenenfalls Katalysatoren in Abwesenheit der oben unter d) angegebenen Ausgangsmaterialien. Ein derartiges Verfahren ist jedoch nicht Gegenstand der vorliegenden Erfindung.

Die europäischen Patentanmeldungen 80 102 171.8 (0 018 581) und 80 102 179.1 (0 018 583) befassen sich mit der Herstellung von Aryl-mono- und/oder -polyurethanen durch Umsetzung von gegebenenfalls substituierten O-Alkyl-, O-Cycloalkyl- oder O-Aralkylcarbamidsäureestern mit primären aromatischen Mono- und/oder Polyaminen in Gegenwart von Alkoholen und gegebenenfalls Harnstoff in Abwesenheit der oben unter d) genannten Ausgangsmaterialien, wobei beim Verfahren der Anmeldung 80 102 179.1 bestimmte Katalysator mitverwendet werden. Auch diese Verfahren sind nicht Gegenstand der vorliegenden Erfindung.

Als Substituenten für die aliphatischen oder cycloaliphatischen Reste $R_1$ bzw. $R_2$ kommen beispielsweise $C_6-C_{10}$-Aryloxy-, $C_1-C_6$-Alkoxy-, $C_1-C_6$-Alkoxy-$C_2-C_4$-alkoxy-, $C_1-C_6$-Acyl-, $C_1-C_6$-Alkylmercapto-, $C_6-C_{10}$-Arylmercapto-, $C_1-C_{12}$-Alkyl-carbonyl-, Bis-($C_1-C_8$-Alkyl)-amino, $C_1-C_6$-Acylamino, Nitro-, Cyano- oder Rhodanoreste in Betracht.

Als Substituenten für die aromatischen oder araliphatischen Reste $R_1$ bzw. $R_2$ kommen neben diesen Substituenten beispielsweise auch $C_1-C_{12}$-Alkyl-, $C_1-C_{12}$-Alkylsulfonyl-, $C_6-C_{10}$-Arylsulfonyl-, $C_1-C_{12}$-Alkylsulfonsäureester-, oder Sulfonamidreste in Betracht.

Bevorzugte erfindungsgemäße Verfahrensprodukte sind jedoch solche der genannten allgemeinen Formel, für welche

$R_1$ für einen aliphatischen Kohlenwasserstoffrest mit 3 bis 18 Kohlenstoffatomen, einen cycloaliphatischen Kohlenwasserstoffrest mit 6 bis 15 Kohlenstoffatomen oder einen gegebenenfalls Methyl-, Methoxy- und/oder Chlor-substituierten und gegebenenfalls Methylenbrücken aufweisenden aromatischen Kohlenwasserstoffrest mit 6 bis 15 Kohlenstoffatomen steht,

$R_2$ für einen $C_1-C_4$-Alkoxy- oder $C_1-C_4$-Alkoxy-$C_2-C_4$-alkoxy-substituierten oder unsubstituierten aliphatischen Kohlenwasserstoffrest mit 1 bis 18, insbesondere 1 bis 4 Kohlenstoffatomen steht, wie er durch Entfernung der Hydroxylgruppe aus einem einerwertigen unsubstituierten primären oder sekundären aliphatischen Alkohol erhalten wird oder für einen Cyclohexyl- oder 2-Phenyl-ethyl-Rest steht und 1 oder 2 bedeutet.

Ausgangsmaterialien für das erfindungsgemäße Verfahren sind

a) primäre Amine der Formel

$R_1(NH_2)_n$

in welcher

$R_1$ und n die bereits genannte Bedeutung haben.

Beispiele geeigneter Amine sind Methylamin, Ethylamin, Propylamin, Isopropylamin, Butylamin, iso-Butylamin, tert.-Butylamin, Hexylamin, Dodecylamin, 2-Ethylhexylamin, Tetradecylamin, Hexadecylamin, Octadecylamin, Allylamin, 1,4-Diaminobutan, 1,6-Diaminohexan, 2,5-Dimethyl-2,5-hexandiamin, Trimethylhexamethylendiamin, 2-Methoxyethylamin, 3-Ethoxypropylamin, 3-Butoxypropylamin, 1,4-Butandiol-bis-(3-aminopropylether), 3-Amino-propansäure-2-methylpropylester, 6-Amino-hexanitril, Lysinester, 11-Aminoundecansäureester, Cyclohexylamin, Trimethylcyclohexylamin, 2-Norbornylmethylamin, Anilin, o,m,p-Chloranilin, 2,3-, 2,4-, 2,5-, 2,6-Dichloranilin, 3,4-Dichloranilin, p,o-Nitroanilin, m,o,p-Tolylamin, 3-Trifluormethylanilin, 3-Chlor-4-methylanilin, Benzylamin, Phenyl-cyclohexylamin, Naphthylamin, 1,4-Diaminocyclohexan, 2,4-, 2,6-Diamino-1-methylcyclohexan, 5-Amino-1-aminomethyl-1,3,3-trimethylcyclohexan, 4,4'-Diaminodicyclohexylmethan, 4,4'-Diamino-3,3'-dimethyl-dicyclohexylmethan, 1,3-Diaminobenzol, 1,4-Diaminobenzol, 2-Chlor-1,4-diaminobenzol, 2,4-Diaminotoluol, 2,6-Diaminotoluol (und Gemische mit 2,4-), 2-(N-Ethyl-amino)-4-aminotoluol, 1,3-Bisaminomethylbenzol, 1,3-Bisaminomethyl-4,6-dimethylbenzol, 1,3-Diamino-2,6-(4,6)-diethyl-4-methylbenzol, 1,3-Diamino-2,4,6-triisopropylbenzol, 1,5-Diamino-naphthalin, 2,7-Diamino-naphthalin, Benzidin, 3,3'-Dichlorbenzidin, 4,4'-Diaminodiphenylmethan (und Rohwaren), 3,3'-Dichlor-4,4'-diamino-diphenylmethan, 2,2-Bis-(4-aminophenyl)-propan, 4,4',4''-Triamino-triphenylmethan, 4,4'-Diaminodiphenylether, 4,4',4''-Triaminotriphenylthiophosphat, p-Methoxyanilin, p-Ethoxyanilin, 1-(4-Chlorphenoxy)-4-aminobenzol, 2,4-Diaminodiphenylether, m-Aminobenzoesäureester, p-Aminobenzorsäureester, 3,5-Diamino-2-methyl-diphenylmethan, 3,5-Diamino-4-methyl-diphenylmethan (und Gemische), 3,5-Diamino-4-methyl-dicyclohexylmethan, 3,5-Diamino-2-methyl-dicyclohexylmethan (und Gemische), 3,5,4'-Triamino-4-methyl-diphenylmethan, 3,5,4'-Triamino-2-methyl-diphenylmethan, 3,5,2'-Triamino-4-methyl-diphenylmethan, 3,5,2'-Triamino-2-methyl-diphenylmethan (und Gemische), 3,5,4'-Triamino-4-methyl-dicyclohexylmethan, 3,5,4'-Triamino-2-methyl-dicyclohexylmethan, 3,5,2'-Triamino-4-methyl-dicyclohexylmethan, 3,5,2'-Triamino-2-methyl-dicyclohexylmethan (und Gemische), Dibenzofuranamin, 1-Aziridinpropanamin, 4-Pyridinmethanamin, 2-Pyridinamin, 1-(3-Aminophenyl)-3-methyl-5-pyrazolon, Pyrimidinamin, N-Aminomorpholin, 2-Aminobenzthiazol.

Besonders bevorzugte Amine sind Propylamin, Isopropylamin, n-Butylamin, sec.-Butylamin, tert.-Butylamin, Stearylamin, Hexamethylendiamin, Cyclohexylamin, 3,3,5-Trimethyl-5-aminomethylcyclohexylamin, 4,4'-Diamino-dicyclohexylmethan, Anilin, p-Chloranilin, 3,4-Dichloranilin, m-Tolylamin, p-Methoxyanilin, 2,4-Diaminotoluol, 2,6-Diaminotoluol, 4,4'-Diaminodiphenylmethan, 2,4'-Diaminodiphenylmethan oder technische Gemische der genannten Diaminotoluole bzw. Diaminodiphenylmethane.

b) Alkohole der Formel

$R_2-OH$

in welcher

$R_2$ die bereits genannte Bedeutung hat.

Beispiele geeigneter Alkohole sind Methanol, Ethanol, Propanol, iso-Propanol, Butanol, iso-Butanol, Pentanol, iso-Pentanol, Hexanol, iso-Hexanol, Heptanol, iso-Heptanol, Octanol, iso-Octanol, Nonanol, iso-Nonanol, Decanol, iso-Decanol, Dodecanol, 2-Ethylhexanol, β-Chlorethanol, 2-Ethylbutanol, Hexadecanol, Octadecanol, Fettalkohol-Gemische, 2-Methoxyethanol, 2-Ethoxyethanol, 2-Propoxyethanol, 2-Butoxyethanol, 2-(2-Methoxyethoxy)-ethanol, 2-(2-Ethoxyethoxy)-ethanol, 2-(2-Butoxyethoxy)-ethanol, Cyclopentanol, Cyclohexanol, Methylcyclohexanol (und Gemisch), Cyclohexamethanol, 3,3,5-Trimethylcyclohexanol, 4-tert.-Butylcyclohexanol, 2-Hydroxydecalin, Borneol, Isoborneol, 1-(2-Hydroxyethoxy)-4-nitrobenzol, Benzylalkohol, 2-Phenylethanol, 2-(Methoxyphenoxy)-ethanol (Gemisch), 1-Phenylethanol, 3-Phenyl-1-propanol, 4-Methoxybenzylalkohol.

Besonders bevorzugte Alkohole sind Methanol, Ethanol, n-Propanol, iso-Propanol, n-Butanol, iso-Butanol, Cyclohexanol, n-Hexanol, 2-Ethylhexanol, β-Phenylethanol, Glykolmonomethylester, Glykolmonoethylether, Diglykolmonomethylether.

c) N-unsubstituierte Urethane der Formel

$R_3-O-CO-NH_2$

in welcher

$R_3$ der Definition von $R_2$ entspricht, wobei $R_2$ und $R_3$ gleiche oder verschiedene, vorzugsweise jedoch gleiche Reste bedeuten. Außerdem kann $R_3$ auch noch für einen, gegebenenfalls chlor- oder $C_1-C_4$-alkylsubstituierten aromatischen Kohlenwasserstoffrest mit insgesamt 6 bis 15 Kohlenstoffatomen stehen.

Beispiele geeigneter N-unsubstituierter Urethane sind Methyl-, Ethyl-, n-Propyl-, iso-Propyl-, n-Butyl-, n-Decyl-, n-Octadecyl-, Cyclohexyl-, Benzyl-, Phenyl-, 4-Chlorphenyl-, 4-Methylphenyl-, 1-Naphthyl- oder tert.-Butyl-carbamat, oder auch die entsprechenden, sich aus den oben genannten Alkoholen b) ableitenden Carbamate. Besonders gut geeignet und daher bevorzugt sind Methyl-, Ethyl-, n-Propyl-, iso-Propyl-, n-Butyl-, iso-Butyl-, Cyclohexyl- oder n-Hexylcarbamat.
Neben diesen wesentlichen erfindungsgemäßen Ausgangsmaterialien a) bis c) können beim erfindungsgemäßen Verfahren auch zusätzlich

d) N-mono- oder N,N'-disubstituierte Harnstoffe bzw. Polyharnstoffe als organische Carbonylverbindungen mitverwendet werden. Es handelt sich hierbei um gegebenenfalls Urethan- oder primäre Aminoendgruppen aufweisende N-mono- oder N,N'-disubstituierte Harnstoffe oder lineare Polyharnstoffe, vorzugsweise eines maximalen Molekulargewichts von 2000, deren Harnstoff-, Urethan- bzw. Amino-Gruppen über Kohlenwasserstoffreste miteinander verknüpft sind oder deren Harnstoffgruppen mit Kohlenwasserstoffresten substituiert sind, die vorzugsweise dem bzw. den Kohlenwasserstoffresten der Amine a) entsprechen, und deren gegebenenfalls vorliegenden endständigen Urethangruppen am Sauerstoffatom vorzugsweise mit dem dem als Ausgangsmaterial eingesetzten Alkohol entsprechenden Rest $-R_2$ substituiert sind, und deren Gesamtgehalt an Harnstoffgruppen $NH-CO-NH-$ und Urethangruppen $-NH-CO-O-$ einem Gehalt an für beide Gruppen charakteristischen Struktureinheiten $NH-CO-$ zwischen 5 und 58 Gew.-%, vorzugsweise zwischen 10 und 58 Gew.-% entspricht.

Typische Beispiele derartiger Harnstoffe bzw. Polyharnstoffe sind N-Methyl-Harnstoff, N,N'-Dimethylharnstoff, N-ethylharnstoff, N-Phenylharnstoff, N,N'-Diphenylharnstoff, N,N'-Bis-(3-methyl-phenyl-)harnstoff, N,N'-Bis-(3,4-dichlorphenyl)-Harnstoff, N,N'-Di-isobutylharnstoff oder Verbindungen der Formeln

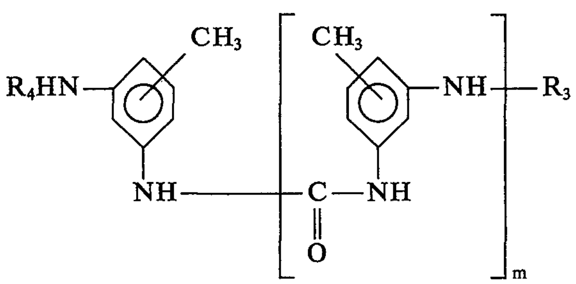

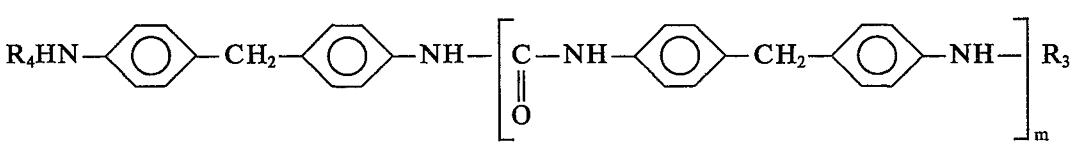

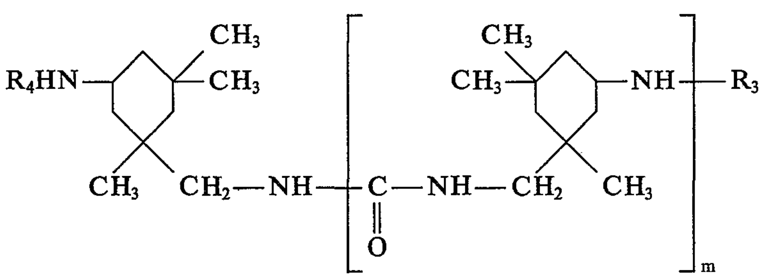

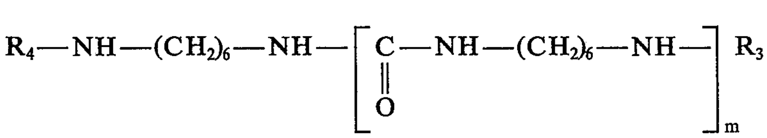

wobei

m eine ganze oder gebrochene (bei statistischen Gemischen) Zahl von 1 bis 0 ist,
$R_4$ und $R_5$ für gleiche oder verschiedene Reste stehen und jeweils H, $COOR_2$, $CONH_2$, $CONHR_6$ bedeuten, wobei $R_6$ für einen einwertigen Rest der bei der Definition von $R_1$ genannten Art steht.

Die N,N'-disubstituierten Monoharnstoffe und die Bis-harnstoffe werden bevorzugt eingesetzt. Geeignete Harnstoffe (e) sind auch die N,N'-disubstituierten Harnstoffe, die bei den Verfahren der US-Patentschriften 2 409 712 und 2 806 051 als Nebenprodukte anfallen oder beliebige gemäß Russ. Chem. Rev. 31 633 (1963) oder Houben-Weyl XIV/2, 165 ff hergestellten Verbindungen.

Bei der Durchführung des erfindungsgemäßen Verfahrens kommt die Amin-Komponente a) inklusive dem gegebenenfalls in der Harnstoff-Komponente d) chemisch gebundenen Amin, falls dieser der Amin-Komponente a) entspricht, im allgemeinen in eine 0,5—4fach, vorzugsweise 0,8—1,5fach und insbesondere 0,9—1,1fach stöchiometrischen Menge und die Alkohol-Komponente b) inklusive dem in der Komponente c) und gegebenenfalls in der Komponente d) chemisch gebundenem Alkohol, falls dieser der Alkoholkomponente b) entspricht, im allgemeinen in einer 1—10fach, vorzugsweise 1,1—4fach stöchiometrischen Menge, jeweils bezogen auf die in der Komponenten c) und d) innerhalb von Harnstoff- oder Urethangruppen vorliegenden Carbonylgruppen zum Einsatz. Die gegebenenfalls mitzuverwendenden Harnstoffverbindungen d) gelangen im allgemeinen in Mengen von 0 bis 300, vorzugsweise 0 bis 200 Gew.-%, bezogen auf die Menge der Komponente c), zum Einsatz.

Das erfindungsgemäße Verfahren wird vorzugsweise unter Mitverwendung geeigneter Katalysatoren e) durchgeführt. Geeignete Katalysytoren sind alle beliebigen Verbindungen, die einen katalytischen Einfluß auf die Veresterungsreaktion von Carbonsäure mit Alkoholen ausüben. Besonders gut geeignete Katalysatoren sind (i) unter den Reaktionsbedingungen inerte organische oder schwache anorganische Basen, (ii) Lewis-Säuren und (iii) Salze bzw. Komplexverbindungen, insbesondere Chelate von Übergangsmetallen.

Beispiele geeigneter Katalysatoren der Gruppe (i) sind tert.-Amine, wie Tri-n-propylamin, Triethylamin, Triisopentylamin, Diethylbenzylamin, N,N-Dimethyl-benzylamin, Hexahydrodimethylanilin, N-Ethylpiperazin, Diethyl(2-methoxypropyl)-amin, 2-(Diethylammoethyl)-phenylether, N-(2-Diethylaminoethyl)-benzamid, N-(2-Diethylaminoethyl)-propionamid, 1,4-Diaza-(2,2,2)-bicyclooctan, N,N-Dimethyl-4-aminopyridin, 1-Azabicycloheptane, 1-Azabicyclooctane, gesättigte polyheterocyclische Amine, wie 3-Methylconidin, 1-Azabicyclo-(3,2,1)-octan, Pyrrolizidine und Chinuclidine, anorganische Basen wie Berylliumhydroxid, Zinkoxid, Magnesium-, Barium- oder Calciumhydroxid, basische Alkalisalze wie Natriumcarbonat, Natriumsulfid, Kaliumcarbonat oder Trinatriumphosphat sowie Alkalisalze von Fettsäuren oder Sulfonsäuren.

Geeignete Katalysatoren (ii) sind beispielsweise Lewis-Säuren wie Eisen-II-chlorid, Eisen-III-chlorid, Zinkchlorid, Zinn-II-chlorid, Zinn-IV-chlorid, Aluminiumchlorid, Zinkcyanid, Bortrifluorid oder Bortrifluoridetherat.

Geeignete Katalysatoren der Gruppe (iii) sind beispielsweise Salze von Übergangsmetallen, soweit sie nicht bereits in die Gruppe (ii) fallen, sowie Komplexverbindungen insbesondere Chelate dieser Metalle wie Kobalt-, Mangan-, oder Bleinaphthenate, Eisenoleate oder -carbonyle, Acetylacetonate von Eisen, Nickel, Kobalt, Zink, Blei, Aluminium, Mangan, Magnesium, Molybdän, Titan, Torium, Zirkon oder Vanadium, Bis-(dibenzoylmethan)-kupfer, Bis-(ethylacetoacetat)-kupfer, -eisen, Koordinationsverbindungen von Titan, Zirkon, Hafnium, Thorium und Mangan mit $\beta$-Diketonen, $\beta$-Ketoestern und $\beta$-Hydroxyal-Di-(2-ethylhexyl)-zinnoxid, Dioctylzinnoxid, Zink- oder Zinnsalze von $C_1-C_{20}$-Carbonsäuren wie Zink- oder Zinn(II)-naphthenat, -hexoat, -palmitat, -stearat oder -dimethylvalerat, Acetate, Chloride, oder Octoate des zwei- oder dreiwertigen Cobalts, des ein- oder zweiwertigen Kupfers oder des zweiwertigen Bleis.

Besonders gut geeignete Katalysatoren sind beispielsweise Zinkchlorid, Zinkacetat, Zinkoctoat, Zinkoxid, Zinkcyanid, Zinn-II-chlorid, Zinn-IV-chlorid, Dibutylzinndilaurat, Kobalttriacetat, Kobalttrichlorid, Kobalttrioctoat, Kupfer(II)-acetat, Kupfer-(I)-chlorid, Kupfer-(II)-sulfat, Bleiacetat oder Bleichlorid. Die Menge des jeweils eingesetzten Katalysators liegt im allgemeinen zwischen 1 ppm und 20 Gew.-%, bevorzugt zwischen 100 ppm und 5 Gew.-%, bezogen auf die Summe der Ausgangsmaterialien a), b), c) und d). Man wird in der Praxis selbstverständlich bestrebt bleiben, die Konzentration der Katalysatoren möglichst gering zu halten. Die optimale Konzentration hängt von der Art der Ausgangsmaterialien und der Aktivität des jeweiligen Katalysators ab und kann in einem einfachen Vorversuch bestimmt werden.

Die Durchführung des erfindungsgemäßen Verfahrens kann sowohl unter Druck als auch drucklos erfolgen. Die Anwendung von Druck beispielsweise im Bereich von 1 bis 80 bar ist oft dann sinnvoll, wenn die Reaktionstemperatur oberhalb dem Siedepunkt einer oder mehrerer der Ausgangsmaterialien liegt. Auch bei Verwendung von niedrigsiedenden Alkoholen ist man nicht unbedingt auf eine Durchführung der Umsetzung unter Druck angewiesen, da man auch in der Weise vorgehen kann, daß man die Reaktionspartner a), c) und d) auf die Reaktionstemperatur erhitzt und den niedrigsiedenden Alkohol so langsam zugibt, daß die Reaktionstemperatur erhalten bleibt. Das erfindungsgemäße Verfahren wird im Temperaturbereich von 120°C bis 350°C, vorzugsweise 130 bis 300°C und

insbesondere 140° C bis 250° C durchgeführt.

Das erfindungsgemäße Verfahren kann in Gegenwart oder auch in Abwesenheit von Lösungsmitteln durchgeführt werden. Geeignete Lösungsmittel sind beispielsweise unter den Verfahrensbedingungen inerte Lösungsmittel mit einem zwischen 100° C und 280° C, vorzugsweise zwischen 150° C und 250° C liegenden Siedepunkt. Beispiele geeigneter Lösungsmittel sind n-Nonan, n-Butylcyclohexan, Decahydronaphthalin, n-Undecan, n-Dodecan, n-Hexylcyclohexan, Dipenten, 1-Dodecen, Isopropylbenzol, 1,3-Diethylbenzol, Inden, n-Butylbenzol, Tetralin, Chlorbenzol, 4-Chlortoluol, 1,2-Dichlorbenzol, 2,4-Dichlortoluol, 1,2,4-Trichlorbenzol, 2-Chlor-4-isopropyl-1-methylbenzol, Anisol, Cyclohexylethylether, Diethylenglykoldimethylether, Benzylmethylether, 4-Methoxytotuol, Parachloranisol, Di-n-hexylether, Phenyl-n-propylketon, Benzophenon, Acetophenon, Formamid, N,N-Dimethylformamid, N,N-Diethylformamid, N-Methylformamid, Dimethylacetamid, N-Methylpyrrolidon, Caprolactam, Phenol, substituierte Phenole, Sulfolan, Hexamethylphosphorsäuretriamid, Dimethylsulfoxid, Ethylenglykolmonoethyletheracetat, Di-n-propylcarbonat, Cyclohexylacetat, Diisobutylcarbonat, Diethylenglykolmonomethyletheracetat, Diisoamylcarbonat, 2-Ethylpyridin, N,N-Dimethyl-2-methylanilin, N,N-Dimethylanilin, N-Methyl-N-ethylanilin; N,N-Dimethyl-2-chloranilin, N,N-Diethylanilin, Chinolin, Nitrocyclohexan, Nitrobenzol, 2-Nitrotoluol, 2,4-Dimethyl-1-nitrobenzol, Acetonitril, N-Capronitril, Benzonitril, Tolunitril, Diphenylether, Tetramethylharnstoff und Phenylacetonitril, besonders bevorzugt sind polare Lösungsmittel und deren Gemische, insbesondere ε-Caprolactam.

Die Mitverwendung derartiger Lösungsmittel ist oft, beispielsweise bei Verwendung eines hohen Überschusses an Alkohol (Komponente b) überflüssig. Insbesondere erübrigt sich die Verwendung derartiger Hilfslösungsmittel bei der Herstellung von Monourethanen ($n=1$), d. h. bei der ausschließlichen Verwendung von Monoaminen als Komponente a).

Zur Durchführung des erfindungsgemäßen Verfahrens werden die Reaktionspartner im allgemeinen während eines Zeitraumes von 1 bis 15, vorzugsweise 2 bis 10 Stunden auf die genannte Reaktionstemperatur erhitzt, wobei man die Komponenten zu Beginn als Gemisch einsetzen kann, oder wobei man auch beispielsweise so vorgehen kann, daß man das Gemisch aus den Komponenten b), c) und gegebenenfalls d) vorlegt, auf die gewünschte Reaktionstemperatur erhitzt und anschließend die Komponente a) hinzufügt.

Bei der Durchführung des erfindungsgemäßen Verfahrens ist es auch möglich, das eingesetzten N-unsubstituierte Urethan c) nur als Carbonylgruppenspender zu verwenden. Falls die Reaktion mit einem Alkohol b) durchgeführt wird, der mit der Alkoholkomponente des Urethans c) nicht identisch ist und insbesondere einen höheren Siedepunkt als diese Alkoholkomponente aufweist, wird bei der Durchführung des erfindungsgemäßen Verfahrens im allgemeinen der im Urethan c) vorliegende Alkohol verdrängt und laufend durch Destillation entfernt, so daß man als Verfahrensprodukt ein disubstituiertes Urethan erhält, dessen Alkoholkomponente ausschließlich dem als Ausgangsmaterial und vorzugsweise auch als Lösungsmittel eingesetzten Alkohol b) entspricht. Auch im Falle der Verwendung von aromatischen N-unsubstituierten Urethanen c) der beispielhaft genannten Art wird vorzugsweise unter Verwendung überschüssiger Mengen an Alkohol b) gearbeitet, wobei auch hier eine Verdrängung des Phenols durch den Alkohol stattfindet, ohne daß hierzu eine laufende Entfernung des sich bildenden Phenols erforderlich wäre. Bei diesen Varianten des erfindungsgemäßen Verfahrens kann es oft zweckmäßig sein, die Verdrängung des dem Urethan c) zugrundeliegenden Alkohols bzw. Phenols durch den Alkohol b) vor der Zugabe des Amins a) durchzuführen.

Bei allen Varianten des erfindungsgemäßen Verfahren muß dafür Sorge getragen werden, daß sich der bildende Ammoniak entweichen kann.

Die Aufarbeitung der erfindungsgemäßen Verfahrensprodukte erfolgt in an sich bekannter Weise, insbesondere durch Abdestillieren von Lösungsmittel und gegebenenfalls vorliegenden überschüssigen flüchtigen Ausgangsmaterialien, gegebenenfalls nach Abfiltrieren von unlöslichen Bestandteilen, wie z. B. unlöslichen Katalysatoren. Die Verfahrensprodukte fallen hierbei im allgemeinen als letzte Fraktion oder als Destillationsrückstand an und können ohne weitere Aufarbeitung in an sich bekannter Weise thermisch in das ihnen zugrundeliegende Isocyanat und den ihnen zugrundeliegenden Alkohol gespalten werden.

In den nachfolgenden Beispielen beziehen sich alle Prozentangaben auf Gewichtsprozente.

## Beispiel 1

Ein Druckbehälter aus Stahl (Nenninhalt 5 l, zulässiger Höchstdruck 64 bar), der mit einem Rührer und einer Mantelheizung versehen ist, wird so mit einer Druckdestillationskolonne aus Stahl verbunden, daß der Behälter als Sumpfgefäß der Kolonne fungiert. Die Kolonne (Nennweite 50 mm) ist mit Füllringen aus Maschendrahtgewebe (4 mm; Stahl) gefüllt (Füllhöhe ca. 1 m) und ist am Kopf mit einem Schlangenkühler als Kondensator versehen. Oberhalb des Kopfkondensators befindet sich ein Ventil zur Entnahme von Gasen aus der Kolonne.

In dieser Apparatur werden 648 g 3,4-Dichloranilin, 360 g Methylcarbamat, 280 g N,N'-Bis-(3,4-dichlorphenyl)-harnstoff, 385 g Methanol und 1600 g Chlorbenzol unter Ammoniak-Entnahme 6,0

Stunden bei 200° C umgesetzt. Nach dem Abkühlen und Entspannen der Apparatur wird das Reaktionsgemisch entnommen, filtriert und mittels Hochdruckflüssigkeitschromatographie (HPLC) analysiert. Nach Eichung mit einer authentischen Substanz wird eine Ausbeute von 990 g (80% der Theorie) N-(3,4-Dichlorphenyl)-carbamidsäure-methylester ermittelt.

Beispiel 2

In die im Beispiel 1 beschriebene Apparatur werden 275 g eines Polyharnstoffgemisches auf Basis von 2,4-Diaminotoluol mit Aminotolyl-Endgruppen (mittleren Molgewicht: 1500) eingefüllt. Dann werden 513 g 2,4-Diaminotoluol, 933 g Ethylcarbamat und 1250 g Ethanol (ca. 96%ig) dazugegeben. Das Gemisch wird unter Ammoniak-Entnahme 6,0 Stunden bei 200° C umgesetzt. Nach dem Abkühlen und Entspannen der Apparatur wird das Reaktionsgemisch entnommen, filtriert und durch Flüssigkeitschromatographie (HPLC) analysiert. Nach Eichung mit einer authentischen Substanz wird eine Ausbeute von 1100 g (68% der Theorie) 2,4-Bis-(ethoxycarbonylamino)-toluol ermittelt.

Beispiel 3

89 g (1 Mol) Carbamidsäureethylester, 93 g (1 Mol) Anilin, 136 g (1 Mol) Phenylharnstoff und 25 g (2,5 Mol) Cyclohexanol wurden mit 0,8 g 2,3,4,6,7,8,9,10-Octahydro-pyrimido-(1,2-a)-azepin am Rückfluß erhitzt. Der Kühler war auf 90° C vorgeheizt, so daß das entstehende Ethanol laufend abdestilliert wurde. Die Temperatur der Reaktionslösung stieg innerhalb von 5 Stunden auf 200° C an. Bei nachlassender Ammoniakentwicklung wurde noch 6 Stunden bei 200° C gerührt. Mit Hilfe der Hochdruckflüssigkeitschromatographie wurde die Ausbeute an O-Cyclohexyl-N-phenylurethan zu 82,5%, bezogen auf eingesetzte Carbonylgruppenäquivalente, bestimmt.

Beispiel 4

301,4 g (2 Mol) Carbamidsäurephenylester, 204,6 g (2,2 Mol) Anilin und 300 g (3 Mol) Cyclohexanol wurden 7 Stunden am Rückfluß erhitzt, wobei bereits nach 4 Stunden die Ammoniakentwicklung fast beendet war. Die Lösung wurde bei 100° C Badtemperatur und 0,3 Torr eingeengt. Im Rückstand wurde mit Hilfe der Hochdruckflüssigkeitschromatographie 72,7% der Theorie O-Cyclohexyl-N-phenylurethan gefunden.

Beispiel 5

In einem 1-l-Rührgefäß werden 133,5 g Carbamidsäureethylester (1,5 Mol) 107 g 3-Amino-toluol (1 Mol), 120 g 3,3'-Dimethyl-N,N'-diphenylharnstoff (0,5 Mol) und 100 g Cyclohexanol (1 Mol) gemischt, mit 2 ml Zinkoctoat versetzt und auf 165° C erwärmt. Die Innentemperatur wird langsam gesteigert und innerhalb von 2 Stunden werden 300 g Cyclohexanol (3 Mol) bei einer Innentemperatur von 180 bis 185° C zugetropft. Innerhalb von 4 Stunden steigt die Innentemperatur auf 194° C an. Der während der Reaktion entstehende Ethylalkohol wird immer direkt abdestilliert.

Zur Aufarbeitung wird das überschüssige Cyclohexanol im Vakuum entfernt, der Rückstand destilliert bzw. aus Waschbenzin umkristallisiert.

| | |
|---|---|
| Ausbeute: | 370 g N-(m-Tolyl)-O-cyclohexylurethan (=79% der Theorie); |
| Sdp.: | 134 bis 138° C bei 0,5 mbar; |
| Fp.: | 82 bis 84° C (aus Waschbenzin). |

Beispiel 6

137 g Carbamidsäurephenylester (1 Mol), 101 g n-Hexylamin (1 Mol), 150 g Cyclohexanol (1,5 Mol) und 2 ml Zinkoctoat werden zügig erwärmt. Bei 155 bis 160° C setzt die $NH_3$-Entwicklung ein. Innerhalb von 2 Stunden wird die Temperatur auf 200° C gesteigert und drei Stunden bei dieser Temperatur gehalten. Anschließend wird das Reaktionsgemisch fraktioniert destilliert.

| | |
|---|---|
| Ausbeute: | 185 g N-(n-Hexyl)-O-cyclohexylurethan (=91% der Theorie); |
| Sdp.: | 130 bis 132° C bei 0,26 Torr. |

Das Produkt wurde durch IR und NMR-Analyse identifiziert.

Beispiel 7

51,8 g der Verbindung

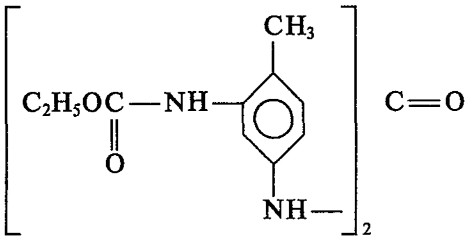

(0,125 Mol), 61 g 2,4-Diaminotoluol (0,5 Mol), 100 g Carbamidsäureethylester (1,125 Mol), 200 g Cyclohexanol (2 Mol) und 3 g Thalliumchlorid werden auf 140° C erwärmt, wobei eine $NH_3$-Entwicklung einsetzt. Der während der Reaktion entstehende Ethylalkohol wird über einen mit Dampf beheizten Kühler abdestilliert. Die Reaktionstemperatur wird auf 200° C gesteigert und 7 Stunden bei dieser Temperatur gehalten. Danach wird noch vorhandenes Cyclohexanol im Wasserstrahlvakuum abdestilliert und der Rückstand mit wenig Essigester versetzt. Über Nacht kristallisiert das Reaktionsgemisch durch.

Ausbeute: 284 g 2,4-Bis-(cyclohexoxycarbonylamino)-toluol (=76% der Theorie);
Fp.: 156° C (aus Toluol).

Beispiel 8

In einem 2-l-Vierhalskolben mit Rührer, Kontaktthermometer, Rückflußkühler und Tropftrichter wurden 234 g (2 Mol) Carbamidsäurebutylester, 186 g (2 Mol) Anilin, 408 g (3 Mol) Monophenylharnstoff, 74 g (1 Mol) n-Butanol und 5 g Titansäuretetrabutylester erhitzt. Ab 130° C begann eine lebhafte Ammoniakentwicklung. Das Gas wurde in verdünnte Schwefelsäure aufgefangen. Unter Zutropfen von 148 g (2 Mol) n-Butanol wurde im Verlauf von 2 Stunden die Temperatur auf 175° C gesteigert. Im Verlauf von weiteren 2 Stunden wurden bei 175° C noch 74 g (1 Mol) n-Butanol zugegeben. Danach waren 4,8 Mol (96% der Theorie) Ammoniak abgespalten. Das Rohprodukt (1020 g) bestand, wie die Hochdruckflüssigkeitschromatographie ergab, zu 86% aus N-Phenyl-O-n-butyl-urethan.

Ausbeute: 91% der Theorie.

Beispiel 9

In einem 2-l-Vierhalskolben mit Rührer, Kontaktthermometer, Rückflußkühler und Tropftrichter wurden 692 g (4 Mol) Carbamidsäure-2-ethylhexylester, 390 g (3 Mol) 2-Ethylhexanol, 344 g (2 Mol) N,N'-Diisobutylharnstoff und 5 g Zinkdioctoat vorgelegt und auf 180° C aufgeheizt. Dabei setzte eine zügige Abspaltung von Ammoniak ein, das in verdünnter Schwefelsäure aufgefangen wurde. Innerhalb von 2 Stunden wurden nun bei 180° C 146 g Isobutylamin zugetropft. Eine Verstärkung der $NH_3$-Abspaltung war die Folge. Im Verlauf von weiteren 3 Stunden wurde die Temperatur allmählich auf 220° C gesteigert. Danach waren 3,8 Mol $NH_3$ (95% der Theorie) entstanden. Der Ansatz (1505 g) wurde geteilt, ein Anteil von 832 g wurde fraktioniert destilliert. Nach einem Vorlauf von im Überschuß eingesetztem 2-Ethylhexanol und einem geringfügigen Zwischenlauf von Carbamidsäure-2-ethylhexylester erhielt man als Hauptfraktion 627 g (2,74 Mol) N-Isobutyl-O-(2-ethylhexyl)-urethan ($Kp_{0,2\,mm}$: 116° C). Die entspricht, bezogen auf den Gesamtansatz, 4,92 Mol oder 82,7% der Theorie.

**Patentansprüche**

1. Verfahren zur Herstellung von Urethanen der allgemeinen Formel

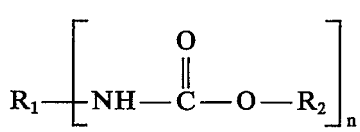

in welcher

$R_1$ für einen gegebenenfalls substituierten aliphatischen Kohlenwasserstoffrest mit 1–18

Kohlenstoffatomen, einen gegebenenfalls substituierten cycloaliphatischen Kohlenwasserstoffrest mit 3 bis 18 Kohlenstoffatomen, einen gegebenenfalls substituierten aromatischen Kohlenwasserstoffrest mit 6–15 Kohlenstoffatomen, einen gegebenenfalls substituierten araliphatischen Kohlenwasserstoffrest mit 7 bis 14 Kohlenstoffatomen, oder für einen gegebenenfalls substituierten 5- oder 6gliedrigen heterocyclischen Rest steht, der zusätzlich noch mit einem Benzolring anelliert sein kann,

$R_2$ für einen gegebenenfalls substituierten Alkylrest mit 1 bis 20 Kohlenstoffatomen, einen gegebenenfalls substituierten Cycloalkylrest mit 3 bis 16 Kohlenstoffatomen und einen gegebenenfalls substituierten Aralkylrest mit 7 bis 14 Kohlenstoffatomen und

n eine ganze Zahl von 1 bis 3 bedeuten,

wobei im Falle von $n=2$ oder 3 zwischen 2 mit dem Rest $R_1$ verknüpften Urethangruppen mindestens 2 Kohlenstoffatome angeordnet sind,

durch Umsetzung von

a) primären Aminen der Formel

$$R_1(NH_2)_n \text{ und}$$

b) Alkoholen der Formel

$$R_2-OH$$

mit Carbonylgruppen aufweisenden organischen Verbindungen im Temperaturbereich von 120 bis 350°C, dadurch gekennzeichnet, daß man als Carbonylgruppen aufweisende organische Verbindungen

c) Urethane der Formel

$$R_3-O-CO-NH_2$$

in welcher

$R_3$ entweder der Definition von $R_2$ entspricht, wobei $R_2$ und $R_3$ gleiche oder verschiedene Reste bedeuten, oder für einen gegebenenfalls Chlor- oder $C_1-C_4$-Alkyl-substituierten aromatischen Kohlenwasserstoffrest mit insgesamt 6 bis 15 Kohlenstoffatomen steht, und gegebenenfalls

d) gegebenenfalls Urethan- oder primäre Aminoendgruppen aufweisende N-mono- oder N,N'-disubstituierte Harnstoffe oder lineare Harnstoffe eines maximalen Molekulargewichts von 2000, deren Harnstoff-, Urethan- bzw. Aminogruppen über Kohlenwasserstoffreste miteinander verknüpft sind oder deren Harnstoffgruppen mit Kohlenwasserstoffresten substituiert sind, die dem Rest $R_1$ entsprechen, und deren gegebenenfalls vorliegende endständigen Urethangruppen mit dem dem als Ausgangsmaterial eingesetzten Alkohol entsprechenden Rest $R_2$ substituiert sind, und deren Gesamtgehalt an Harnstoffgruppen $-NH-CO-NH-$ und Urethangruppen $-NH-CO-O-$ einem Gehalt an für beide Gruppen charakteristischen Struktureinheiten $-NH-CO-$ zwischen 5 und 58 Gew.-% entspricht,

verwendet, wobei sowohl (i) die Herstellung von Aryl-mono- und/oder -polyurethanen durch Umsetzung von gegebenenfalls substituierten O-Alkyl-, O-Cycloalkyl- oder O-Aralkylcarbamidsäureestern mit primären aromatischen mono- und/oder Polyaminen in Gegenwart von Alkoholen ohne gleichzeitige Mitverwendung der unter d) genannten Ausgangsverbindungen als auch (ii) die Herstellung von aliphatischen und/oder cycloaliphatischen Di- und/oder Polyurethanen durch Umsetzung von Carbamidsäureestern mit primären aliphatischen und/oder cycloaliphatischen Di- und/oder Polyaminen und Alkoholen in Abwesenheit der unter d) genannten Ausgangsverbindungen ausgenommen sind.

2. Verfahren gemäß Anspruch 1, dadurch gekennzeichnet, daß man die Umsetzung in Gegenwart von Veresterungskatalysatoren für Carbonsäuren als Katalysatoren durchführt.

3. Verfahren gemäß Anspruch 1 und 2, dadurch gekennzeichnet, daß man die Umsetzung in polaren Lösungsmitteln bzw. Lösungsmittelgemischen durchführt.

4. Verfahren gemäß Anspruch 3, dadurch gekennzeichnet, daß man als Lösungsmittel überschüssige Mengen des als Reaktionspartner zum Einsatz gelangenden Alkohols verwendet.

5. Verfahren gemäß Anspruch 3, dadurch gekennzeichnet, daß man als Lösungsmittel ε-Caprolactam verwendet.

## Claims

1. A process for the production of urethans corresponding to the general formula:

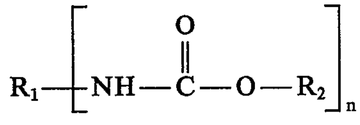

wherein

$R_1$ represents an optionally substituted aliphatic hydrocarbon radical having from 1 to 18 carbon atoms, an optionally substituted cycloaliphatic hydrocarbon radical having from 3 to 18 carbon atoms, an optionally substituted aromatic hydrocarbon radical having from 6 to 15 carbon atoms, an optionally substituted araliphatic hydrocarbon radical having from 7 to 14 carbon atoms, or an optionally substituted 5- or 6-membered heterocyclic radical which may also be anellated with a benzene ring,

$R_2$ represents an optionally substituted alkyl radical having from 1 to 20 carbon atoms, an optionally substituted cycloalkyl radical having from 3 to 16 carbons atoms and an optionally substituted aralkyl radical having from 7 to 14 carbon atoms, and

n represents an integer from 1 to 3,

and if $n=2$ or 3, at least two carbon atoms are positioned between two urethane groups joined to the radical $R_1$,

by the reaction of

a) primary amines corresponding to the formula:

$R_1(NH_2)_n$ and

b) alcohols corresponding to the formula:

$R_2-OH$

with organic compounds containing carbonyl groups in a temperature range of from 120 to 350° C, characterised in that the following are used as organic compounds containing carbonyl groups:

c) urethanes corresponding to the formula:

$R_3-O-CO-NH_2$

wherein

$R_3$ either corresponds to the definition of $R_2$, in which case $R_2$ and $R_3$ represent the same or different radicals, or represents an optionally chlorine- or $C_1-C_4$ alkyl-substituted aromatic hydrocarbon radical having a total of from 6 to 15 carbon atoms, and optionally

d) N-mono- or N,N'-disubstituted ureas optionally containing urethane or primary amino terminal groups, or linear ureas of a maximum molecular weight of 2000, the urea, urethane or amino groups of which are joined together via hydrocarbon radicals, or the urea groups of which are substituted with hydrocarbon radicals which correspond to the radical $R_1$, and the optionally present terminal urethane groups of which are substituted with the radical $R_2$ corresponding to the alcohol which is used as a starting material, and the total content thereof of ureas groups $-NH-CO-NH-$ and urethane groups $-NH-CO-O-$ corresponds to a content of structural units $-NH-CO-$ which is characteristic of both groups, of from 5 to 58% by weight,

and (i) the production of aryl-mono- and/or -polyurethanes by the reaction of optionally substituted O-alkyl-, O-cycloalkyl- or O-aralkylcarbamic acid esters with primary aromatic mono- and/or polyamines in the presence of alcohols without the simultaneous use of the starting compounds mentioned under d), as well as (ii) the production of aliphatic and/or cycloaliphatic di- and/or polyurethanes by the reaction of carbamic acid esters with primary aliphatic and/or cycloaliphatic di- and/or polyamines and alcohols in the absence of the starting compounds mentioned under d) are excluded.

2. A process according to claim 1, characterised in that the reaction is carried out in the presence of

esterification catalysts for carboxylic acids as catalysts.

3. A process according to claims 1 and 2, characterised in that the reaction is carried out in polar solvents or solvent mixtures.

4. A process according to claim 3, characterised in that excess quantities of the alcohol used as the reactant are used as solvent.

5. A process according to claim 3, characterised in that ε-caprolactam ist used as solvent.

**Revendications**

1. Procédé de production d'uréthannes de formule générale:

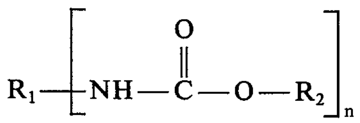

dans laquelle

$R_1$ désigne un reste d'hydrocarbure aliphatique ayant 1 à 18 atomes de carbone éventuellement substitué, un reste d'hydrocarbure cycloaliphatique ayant 3 à 18 atomes de carbone éventuellement substitué, un reste d'hydrocarbure aromatique ayant 6 à 15 atomes de carbone éventuellement substitué, un reste d'hydrocarbure araliphatique ayant 7 à 14 atomes de carbone éventuellement substitué ou un reste hétérocyclique pentagonal ou hexagonal éventuellement substitué qui peut en outre être condensé avec un noyau benzénique,

$R_2$ est un reste alkyle ayant 1 à 20 atomes de carbone éventuellement substitué, un reste cycloalkyle ayant 3 à 16 atomes de carbone éventuellement substitué et un reste aralkyle ayant 7 à 14 atomes de carbone éventuellement substitué, et

n est un nombre entier de 1 à 3,

au moins deux atomes de carbone étant disposé entre deux groupes uréthanne liés au reste $R_1$ au cas où n est égal à 2 ou 3,

par réaction

a) d'aimes primaires de formule

$$R_1(NH_2)_n$$

b) d'alcools des formule

$$R_2-OH$$

avec des composés organiques porteurs de groupes carbonyle dans la plage de températures de 120 à 350°C, caractérisé en ce qu'on utilise comme composés organiques porteurs de groupes carbonyle

c) des uréthannes de formule

$$R_3-O-CO-NH_2$$

dans laquelle

$R_3$ correspond à la définition de $R_2$, auquel cas $R_2$ et $R_3$ désignent des restes égaux ou différents, ou représentent un reste d'hydrocarbure aromatique ayant au total 6 à 15 atomes de carbone, éventuellement substitué avec du chlore ou un radical alkyle en $C_1$ à $C_4$, et, le cas échéant

d) des urées N-mono- ou N,N'-disubstituées ou des urées linéaires portant éventuellement des groupes terminaux uréthanne ou amino primaires d'un poids moléculaire maximal de 2000, dont les groupes urée, uréthanne ou amino sont liés ensemble par l'intermédiaire de restes hydrocarbonés ou dont des groupes urée sont substitués avec des restes hydrocarbonés qui correspondant au reste $R_1$ et dont des groupes uréthanne terminaux éventuellement présents sont substitués avec le reste $R_2$ correspondant à l'alcool utilisé comme matière de départ et dont la teneur totale en groupes urée $-NH-CO-NH-$ et en groupes uréthanne $-NH-CO-O-$ correspond à une teneur en motifs structuraux $-NH-CO-$ caractéristiques des deux groupes entre 5 et 58% en poids,

en exceptant (i) la production d'aryl-mono- et/ou -polyuréthannes par réaction d'esters d'acides O-alkyl-, O-cycloalkyl- ou O-aralkylcarbamiques éventuellement substitués avec des mono- et/ou des polyamines aromatiques primaires en présence d'alcools sans l'utilisation simultanée des composés de départ mentionnés en d) de même que (ii) la production de di- et/ou de polyuréthannes aliphatiques et/ou cycloaliphatiques par réaction d'esters d'acides carbamiques avec des di- et/ou polyamines aliphatiques et/ou cycloaliphatiques primaires et des alcools en l'absence des composés de départ mentionnés en d).

2. Procédé suivant la revendication 1, caractérisé en ce qu'on conduit la réaction en présence de catalyseurs d'estérification pour acides carboxyliques.

3. Procédé suivant les revendications 1 et 2, caractérisé en ce que la réaction est conduite dans des solvants ou des mélanges de solvants polaires.

4. Procédé suivant la revendication 3, caractérisé en ce qu'on utilise comme solvant des quantités en excès de l'alcool utilisé comme partenaire réactionel.

5. Procédé suivant la revendication 3, caractérisé en ce qu'on utilise l'ε-caprolactame comme solvant.